Europäisches Patentamt

European Patent Office

Office européen des brevets

(19)

(11) Veröffentlichungsnummer: **0 023 006**
**B1**

(12) # EUROPÄISCHE PATENTSCHRIFT

(45) Veröffentlichungstag der Patentschrift:
**02.03.83**

(21) Anmeldenummer: **80104055.1**

(22) Anmeldetag: **12.07.80**

(51) Int. Cl.³: **C 07 J 5/00,** C 07 J 7/00,
C 07 J 13/00

(54) **Verfahren zur Herstellung von ungesättigten Steroiden.**

(30) Priorität: **18.07.79 DE 2929558**

(43) Veröffentlichungstag der Anmeldung:
**28.01.81 Patentblatt 81/4**

(45) Bekanntmachung des Hinweises auf die Patenterteilung:
**02.03.83 Patentblatt 83/9**

(84) Benannte Vertragsstaaten:
**BE CH DE FR GB IT LI LU NL SE**

(56) Entgegenhaltungen:
**FR-A-2 108 698**
**FR-A-2 193 818**

(73) Patentinhaber: **SCHERING AKTIENGESELLSCHAFT Berlin und Bergkamen,
Müllerstrasse 170/178 Postfach 65 03 11,
D-1000 Berlin 65 (DE)**

(72) Erfinder: **Schulze, Paul-Eberhard, Dr., Endestrasse 30,
D-1000 Berlin 39 (DE)**
Erfinder: **Kerb, Ulrich, Dr., Prinzregentenstrasse 7,
D-1000 Berlin 31 (DE)**

## Verfahren zur Herstellung von ungesättigten Steroiden

Die Erfindung betrifft den Gegenstand des Patentanspruchs.

Unter Acyloxy sind solche Säurereste zu verstehen, die sich von Alkansäuren mit bis zu 10, vorzugsweise mit bis zu 6 C-Atomen und von aromatischen Säuren mit 7 – 11 C-Atomen ableiten, die gegebenenfalls auch noch durch niederes Alkyl mit z. B. 1 – 4 C-Atomen oder Halogen, wie Chlor oder Jod, substituiert sein können. Genannt seinen beispielsweise die Essig-, Propion-, Isobutter-, Valerian-, Capron- und m-Jodbenzoesäure.

Bekanntermaßen kann man $\Delta^{9(11)}$-ungesättigte Steroide aus der 11$\beta$- oder 11$\alpha$-Alkoholen durch Überführung in die Sulfonate (z. B. Mesylate) und Eliminierung dieser Ester unter basischen Bedingungen (P. Wieland et al., Helv. 43, 523 [1960]) oder nach Schutz der Carbonylgruppen mit Phosphoroxychlorid in Pyridin (S. Bernstein et al., JACS 75, 4830 [1953]) herstellen.

Weiterhin ist bekannt, daß man $\Delta^{16}$-ungesättigte Steroide der Pregnanreihe aus den entsprechenden 17$\alpha$-Hydroxy-20-keteopregnanen durch Behandlung mit Phosphoroxychlorid in Pyridin bei Raumtemperatur erhalten kann (Fried & Edwards, Organic Reactions in Steroid Chemistry, Vol. II, S. 171 [1972]).

Die bekannten chemischen Methoden haben jedoch den Nachteil, daß sie entweder über mehrere Stufen verlaufen oder unbefriedigende Ausbeuten liefern.

Der Verlauf der erfindungsgemäßen Reaktion war insofern überraschend, da zu erwarten war, daß beim Erhitzen auf Temperaturen über den Schmelzpunkt der betreffenden Verbindungen thermische Zersetzungen und Umlagerungen auftreten würden. Es werden jedoch sterisch einheitliche Verbindungen erhalten.

Das erfindungsgemäße Verfahren ist bevorzugt anwendbar auf solche Steroide, die allein in 17$\alpha$-Stellung eine Acyloxygruppe aufweisen, auf solche, die allein in 16-Stellung einen Chlorsubstituenten haben und auf solche, die sowohl in 17$\alpha$-Stellung eine Acyloxygruppe als auch in 9$\alpha$-Stellung einen Chlorsubstituenten aufweisen. Im allgemeinen ist es einfacher, den Chlorsubstituenten in 9$\alpha$- bzw. 16-$\alpha$-Stellung zu eliminieren als die Acyloxygruppe in der 17$\alpha$-Stellung.

Durch Wahl milderer Reaktionsbedingungen, d. h., tiefere Temperatur, z. B. 180 – 240° C, kann die 9(11)-Doppelbindung erhalten werden, während die 16-Stellung gesättigt bleibt, wenn ein 17$\alpha$-Acyloxysubstituent am Molekül vorhanden ist. Gewünschtenfalls kann somit bei weiterer Steigerung der Temperatur, z. B. auf 240 – 300° C, auch die Doppelbindung in 16-Stellung erhalten werden.

Will man sowohl die $\Delta^{9(11)}$- als auch die $\Delta^{16}$-Doppelbindung aus den entsprechenden 9$\alpha$-Chlor-17$\alpha$-acyloxysteroiden gleichzeitig erhalten, muß man die Reaktion entsprechend im höheren Temperaturbereich durchführen.

Die als Ausgangsmaterial verwendeten 17$\alpha$-Acyloxy- bzw. 9$\alpha$- und 16$\alpha$-Chlorsteroide gehören den Steroiden der Pregnanreihe an, wobei die 17$\beta$-ständige Seitenkette auch ungesättigt sein kann. Sie können an sich noch weiter substituiert sein.

Als Substituenten kommen beispielsweise in Frage niedere Alkylgruppen mit z. B. 1 – 4 C-Atomen, wie Methyl in 2-, 6-, 16-, 18- und 21-Stellung, Halogen wie Chlor in 2-, 6- und 12-Stellung und die Hydroxygruppe in 3- oder 11-Stellung. Aber auch Alkinylgruppen, wie die Äthinylgruppe in 17-Stellung, und Ketogruppen können in 3-, 11- und 20-Stellung vorhanden sein. Eine Methylengruppe kann in 1.2- oder 6.7-Stellung stehen.

Doppelbindungen können sich in 1-, 4-, 5- und 6-Stellung befinden. Weitere Acyloxygruppen können in 3- und/oder 21-Stellung auftreten. Die niederen Alkylgruppen und Halogen können sich insbesondere an C-Atomen mit einer Doppelbindung befinden, wie z. B. in der Gruppierung $\Delta^6$-6-Chlor.

Das erfindungsgemäße Verfahren wird so durchgeführt, daß man das Ausgangsmaterial in dem erfindungsgemäß verwendeten Lösungsmittel löst und bei Temperaturen von 180 – 350, vorzugsweise 200 – 300° C über eine Zeit von 5 bis 100 Minuten erwärmt.

Geeignete Lösungsmittel zur Durchführung des erfindungsgemäßen Verfahrens sind inerte aprotische Lösungsmittel mit einem hohen Siedepunkt, wie z. B. 200 – 400° C. Genannt seien z. B. Biphenyl, Diphenylenoxid, Dibenzylbenzol, Oligoglykoldimethylether wie Di-, Tri- und Polyglykol-200-dimethyläther und deren Gemische untereinander. Diese Flüssigkeiten sind z. T. im Handel. Unter dem Namen Dowtherm®A erhält man eine eutektische Mischung von Biphenyl und Dibenzofuran (ca. Kp. 285° C), unter dem Namen Marlotherm®S Dibenzylbenzol-Isomerengemische (ca. Kp. 390° C) unter unter der Bezeichnung Polyglykol-200-dimethyläther ein Homologengemisch von Pentaäthylenglykoldimethyläther $CH_3O(CH_2CH_2O)_nCH_3$, n = 2 – 10 (Siedebereich 240 – 350° C).

Das erfindungsgemäß verwendete Lösungsmittel wird in einer Menge von 2 – 50, vorzugsweise 5 – 20 Gewichtsteilen, bezogen auf die Menge des Ausgangsmaterials, eingesetzt.

Es ist zweckmäßig, das Reaktionsgemisch unter einer Schutzgasatmosphäre, wie z. B. Stickstoff, zu erwärmen, um den Einfluß von Sauerstoff auszuschließen. Vorteilhaft ist auch das feste Eintragen der Substanz unter Schutzgas in das zuvor auf die gewünschte Temperatur gebrachte Lösungsmittel. Der Verlauf der Thermolyse läßt sich leicht dünnschichtchromatographisch verfolgen. Nach beendeter Reaktion wird das Reaktionsgemisch abgekühlt und in üblicher Weise, wie durch Filtrieren, Waschen und Eluieren, aufgearbeitet.

Eine bevorzugte Aufarbeitungsform ist die Entfernung des Lösungsmittels durch Wasserdampfdestillation, Trocknung des Rückstandes und Umkristallisation.

Allgemein gesehen hat das erfindungsgemäße Verfahren den Vorteil, daß es aus einer überaus einfachen Manipulation besteht. Die Substanz wird in dem Lösungsmittel erhitzt und nach der Reaktion wieder von dem Lösungsmittel getrennt.

Ein weiterer Vorteil des Verfahrens besteht darin, daß abgespaltener Chlorwasserstoff bzw. leicht flüchtige organische Säuren aus der Reaktionsflüssigkeit beim Erhitzen entweichen. Eine Neutralisierung ist nicht notwendig. Säurekatalysierte Umlagerungen, wie z. B. die Dienonphenol-Umlagerung von $\Delta^{1.4}$-3-Ketosteroiden, können gar nicht erst auftreten.

Ein weiterer Vorteil gegenüber dem sonst gebräuchlichen Verfahren der Chlorwasserstoff-Eliminierung mittels Silberperchlorat aus 9α-Chlorsteroiden unter Bildung der entsprechenden $\Delta^{9(11)}$-Verbindungen besteht darin, daß man nicht mit gefährlichen Substanzen, wie dem genannten Silberperchlorat, arbeitet.

Die nach dem erfindungsgemäßen Verfahren herstellbaren Verbindungen sind Ausgangsstoffe zur Herstellung von bekannten Wirkstoffen. So kann man beispielsweise aus 3β-Hydroxy-5.16-pregnadien-20-on das Prednisolon sowie nach Einführung einer 16α-ständigen Methylgruppe das Fluocortolon, Clocortolon und Diflucortolon herstellen. Aus den entsprechenden 21-funktionalisierten $\Delta^{16}$-ungesättigten 3.20-Ketopregnenen lassen sich so bekannte Corticoide wie Triamcinolon, Dexamethason und Betamethason herstellen.

Die nachfolgenden Beispiele sollen das erfindungsgemäße Verfahren erläutern.

## Beispiel 1

1 g 17α-Acetoxy-11β-benzoyloxy-1α.2α-methylen-4.6-pregnadien-3.20-dion werden in 10 ml Dowtherm® (über Al₂O₃ filtriert) gelöst. Die Lösung wird unter Stickstoffbegasung 30 Minuten auf 285°C erhitzt. Nach Abkühlen wird mit Hexan verdünnt, auf eine Silicagelsäule gegeben und das Dowtherm® mit Hexan eluiert. Nun wird mit Methylenchlorid und einem graduell ansteigendem Anteil Chloroform die hergestellte Verbindung eluiert. Die Einzelfraktionen werden nach Dünnschichtchromatographie auf ihren Gehalt und Reinheit geprüft. Die Fraktionen mit der gewünschten Substanz werden zusammengefaßt, eingeengt und der Rückstand aus Benzol umkristallisiert. Man erhält 700 mg 11β-Benzoyloxy-1α.2α-methylen-4.6.16-pregnatrien-3.20-dion (79% d. Th.) vom Schmelzpunkt 227 – 229°C.

## Beispiel 2

1 g 17α-Acetoxy-1α.2α-methylen-11β-hydroxy-4.6-pregnadien-3.20-dion werden analog Beispiel 1 in Marlotherm® 60 Minuten auf 285°C erhitzt, aufgearbeitet, getrennt und zusammengefaßt. Man erhält nach Umkristallisieren aus Äthanol 780 mg 1α.2α-Methylen-11β-hydroxy-4.6.16-pregnatrien-3.20-dion (81% d. Th.) vom Schmelzpunkt 245 – 248°C.

## Beispiel 3

1 g 17α-Acetoxy-6-chlor-1α.2α-methylen-4.6-pregnadien-3.20-dion werden analog Beispiel 1 in Dowtherm® 90 Minuten auf 285°C erhitzt, aufgearbeitet, getrennt und zusammengefaßt. Man kristallisiert aus Äthanol um und erhält 380 mg 6-Chlor-1α.2α-methylen-4.6.16-pregnatrien-3.20-dion (89% d. Th.) vom Schmelzpunkt 243 – 246°C.

## Beispiel 4

1 g 17α-Hexanoyloxy-4-pregnen-3.20-dion werden analog Beispiel 1 in Dowtherm® 80 Minuten auf 285°C erhitzt, aufgearbeitet, getrennt und zusammengefaßt. Nach Umkristallisation aus Äthanol erhält man 600 mg an 4.16-Pregnadien-3.20-dion (83% d. Th.) vom Schmelzpunkt 185 – 190°C.

## Beispiel 5

1 g 17α.21-Diacetoxy-4-pregnen-3.20-dion werden analog Beispiel 1 80 Minuten auf 285°C in Dowtherm® erhitzt, aufgearbeitet, getrennt und zusammengefaßt. Man kristallisiert aus Hexan/Essigester um und erhält 700 mg 21-Acetoxy-4.16-pregnadien-3.20-dion (83% d. Th.) vom Schmelzpunkt 146 – 148°C.

## Beispiel 6

1 g 17α-Diacetoxy-5-pregnen-20-on wird analog Beispiel 1 in Dowtherm® 40 Minuten auf 285°C erhitzt und aufgearbeitet. Man kristallisiert aus Äthanol um und erhält 700 mg 3β-Acetoxy-5.16-pregnadien-20-on (80% d. Th.) vom Schmelzpunkt 169 – 172° C.

## Beispiel 7

1 g 17α-Hexanoyloxy-19-nor-4-pregnen-3.20-dion wird analog Beispiel 1 in Dowtherm® 70 Minuten bei 280°C behandelt und aufgearbeitet. Man kristallisiert aus Isopropyläther um und erhält 650 mg 19-Nor-4.16-pregnadien-3.20-dion (93% d. Th.) vom Schmelzpunkt 163 – 166° C.

## Beispiel 8

1 g 21-Acetoxy-17α-(3′-Jodbenzoyloxy)-4-pregnen-3.20-dion wird analog Beispiel 1 in Dowtherm® 15 Minuten bei 250°C behandelt und aufgearbeitet. Man kristallisiert aus Isopropyläther um und erhält 670 mg 21-Acetoxy-4.16-pregnadien-3.20-dion (93% d. Th.).
UV (Methanol) $\varepsilon_{241} = 24\ 000$

## Beispiel 9

1 g 17α.21-Diacetoxy-11β-hydroxy-1.4-pregnadien-3.20-dion wird analog Beispiel 1 in Dowtherm® 50 Minuten bei 250°C behandelt und aufgearbeitet. Man kristallisiert aus Isopropyläther um und erhält 700 mg 21-Acetoxy-11β-hydroxy-1.4.16-pregnatrien-3.20-dion (81% d. Th.).
UV (Methanol) $\varepsilon_{242} = 22\ 500$

## Beispiel 10

1 g 17α-Acetoxy-17β-äthinyl-4-östren-3-on wird analog Beispiel 1 in Dowtherm® 10 Minuten bei 250°C behandelt und aufgearbeitet. Man kristallisiert aus Isopropyläther um und erhält 500 mg 17-Äthinyl-4.16-östradien-3-on vom Schmelzpunkt 148 – 152° C (40% d. Th.).
UV: $\varepsilon_{236} = 23\ 000$

## Beispiel 11

1 g 17α-Acetoxy-12α-chlor-1α.2α-methylen-4.6-pregnadien-3.20-dion wird analog Beispiel 1 in Dowtherm® 1 Stunde bei 280°C behandelt und aufgearbeitet. Nach Umkristallisieren aus Isopropyläther erhält man 500 mg 12α-Chlor-1α.2α-methylen-4.6.16-pregnatrien (61% d. Th.).
UV: $\varepsilon_{235} = 12\ 150$; $\varepsilon_{281} = 15\ 800$

## Beispiel 12

1 g 3β-Acetoxy-16α-chlor-5-pregnen-20-on wird analog Beispiel 1 in Dowtherm® 6 Stunden bei 240°C behandelt und aufgearbeitet. Nach Umkristallisation aus Isopropyläther erhält man 400 mg 3β-Acetoxy-5.16-pregnadien-20-on (56% d. Th.).
UV: $\varepsilon_{239} = 6400$

## Beispiel 13

2,4 g 17.21-Bis-(3-jodbenzoyloxy)-1.4-pregnadien-3.20-dion werden in 15 ml Dowtherm® unter Argon 40 Minuten bei 260°C Ölbadtemperatur gerührt. Nach dem Abkühlen wird mit Methylenchlorid verdünnt, die auskristallisierte Jodbenzoesäure abgesaugt und das Filtrat an Silicagel chromatographiert. Mit Toluol-Nitromethan werden 1,2 g 21-(3-Jodbenzoyloxy)-pregna-1.4.16-trien-3.20-dion (72% d. Th.) eluiert.
Schmelzpunkt: 168 – 169° C.

### Beispiel 14

1,3 g 9α-Chlor-21-acetoxy-17α-(3-jodbenzoyloxy)-4-pregnen-3.20-dion werden in 10 ml Dowtherm® unter Argon bei 220°C Ölbadtemperatur 20 Minuten gerührt, dabei kristallisiert das entstehende Produkt aus. Nach dem Abkühlen wird mit Methylenchlorid verdünnt und an Silicagel chromatographiert. Mit Toluol-Ether wird das 21-Acetoxy-17α-(3-jodbenzoyloxy)-pregna-4.9(11)-dien-3.20-dion eluiert und aus Methylenchlorid-Essigester umkristallisiert.
Schmelzpunkt: 257–260°C. Ausbeute: 79% d. Th.

### Beispiel 15

616 mg 21-Acetoxy-17α-(3-jodbenzoyloxy)-pregna-4.9(11)-dien-3.20-dion werden in 5 ml Dowtherm® 20 Minuten bei 260°C Badtemperatur gerührt und chromatographiert analog Beispiel 1. Man erhält so in 60%iger Ausbeute 21-Acetoxy-pregna-4.9(11).16-trien-3.20-dion vom Schmelzpunkt 128–129°C (Ether).

### Beispiel 16

300 mg 9α-Chlor-17.21-Bis-(3-jodbenzoyloxy)-1.4-pregnadien-3.20-dion werden in 2 ml Dowtherm® 40 Minuten bei 260°C Ölbadtemperatur gerührt und chromatographiert. Man erhält so das 21-(3-Jodbenzoyloxy)-1.4.9(11).16-pregna-tetraen-3.20-dion vom Schmelzpunkt 151–152°C (Aceton-Hexan).
Ausbeute: 74% d. Th.

### Beispiel 17

1,3 g 9α-Chlor-21-acetoxy-17α-(3-jodbenzoyloxy)-4-pregnen-3.20-dion werden in 10 ml Dowtherm® unter Argon 15 Minuten bei 280°C Ölbadtemperatur gerührt. Nach Chromatographie an Silicagel erhält man 518 mg 21-Acetoxy-pregna-4.9(11).16-trien-3.20-dion (70% d. Th.) vom Schmelzpunkt 127–129°C.

### Beispiel 18

Analog Beispiel 1 werden die folgenden Verbindungen unter den angegebenen Reaktionsbedingungen hergestellt:

|   | Endprodukt | Ausgangsmaterial | Temp. [°C] | Zeit [min] | Ausbeute [%] |
|---|---|---|---|---|---|
| a | 11β-Hydroxy-21-acetoxy-pregna-1.4.16-trien-3.20-dion | 11β-Hydroxy-17α-21-diacetoxy-pregna-1.4-dien-3.20-dion | 250 | 50 | 78 |
| b | 3β-Hydroxy-pregna-5.16-dien-20-on | 3β-Hydroxy-17α-acetoxy-5-pregnen-20-on | 285 | 40 | 74 |
| c | 3.20-Diketo-pregna-4.16-dien | 17α-Acetoxy-4-pregnen-3.20-dion | 285 | 50 | 80 |
| d | 11β-Hydroxy-pregna-4.6.16-trien-3.20-dion | 11β-Hydroxy-17α-acetoxy-pregna-4.6-dien-3.20-dion | 285 | 90 | 88 |

### Beispiel 19

1,19 g 9α-Chlor-17α-(3-jodbenzoyloxy)-4-pregnen-3.20-dion werden in 10 ml Polyglykol-200-dimethyläther 20 Minuten bei 280°C Ölbadtemperatur unter Argon gerührt. Nach dem Abkühlen wird in eine eiskalte Natriumhydrogencarbonat-Lösung gegossen, das ausgefällte Produkt abgesaugt und getrocknet. Nach Umkristallisation aus Methanol erhält man 580 mg 4.9(11).16-Pregnatrien-3.20-dion vom Schmelzpunkt 200–201°C (Ausbeute: 93% d. Th.).

### Beispiel 20

5,95 g 9α-Chlor-17α-(3-jodbenzoyloxy)-4-pregnen-3.20-dion werden in 30 ml Dowtherm® bei einer Ölbadtemperatur von 280° C unter Argonbegasung 20 Minuten gerührt. Nach Filtration über Silicagel und Umkristallisation aus Methanol erhält man 2,92 g 4.9(11).16-Pregnatrien-3.20-dion vom Schmelzpunkt 200,5 – 201,5° C.
(Ausbeute: 94% d. Th.)

## Patentanspruch

Verfahren zur Herstellung von $\Delta^{9(11)}$- und/oder $\Delta^{16}$-ungesättigten Steroiden aus 9α- und 16α-Chlor- bzw. 17α-Acyloxysteroiden der Pregnanreihe, dadurch gekennzeichnet, daß man das Ausgangssteroid in einem inerten, aprotischen hochsiedenden Lösungsmittel auf 180 – 350, vorzugsweise 200 – 300° C erhitzt.

## Claim

Process for the preparation of $\Delta^{9(11)}$- and/or $\Delta^{16}$-unsaturated steroids of the pregnane series, characterised in that the corresponding 9α-chloro and 16α-chloro or respectively 17α-acyloxy steroid is heated in an inert, aprotic, high boiling solvent at a temperature of 180 – 350° C., preferably of 200 – 300° C.

## Revendication

Procédé de préparation de stéroïdes insaturés contenant une double liaison $\Delta^{9(11)}$ et/ou une double liaison $\Delta^{16}$ à partir de chloro-9α- et chloro-16α ou acyloxy-17α stéroïdes de la série du prégnane, procédé caractérisé en ce qu'on chauffe le stéroïde de départ, dans un solvant aprotique inerte à haut point d'ébullition, à une température de 180 à 350° C, de préférence, de 200 à 300° C.